# FASCICULE DE BREVET EUROPEEN

(11) **EP 0 828 554 B1**
(45) Date de publication et mention de la délivrance du brevet: **04.07.2001**
(21) Numéro de dépôt: 96917546.2
(22) Date de dépôt: 24.05.1996
(51) Int. Cl.: B01F 17/00, A61K 7/48, B01F 17/56

(54) **COMPOSITION EMULSIONNANTE A BASE D'ALKYLPOLYGLYCOSIDES, ET SES UTILISATIONS**
EMULGIERENDE ZUSAMMENSETZUNG AUF BASIS VON ALKYLPOLYGLUCOSIDEN UND DEREN VERWENDUNGEN
ALKYLPOLYGLYCOSIDES-BASED EMULSIFYING COMPOSITION AND USES THEREOF

(30) Priorité: 24.05.1995 FR 9506234
(43) Date de publication de la demande: 18.03.1998
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: AMALRIC, Chantal, F-81700 Blan (FR); MICHEL, Nelly, F-94700 Maisons-Alfort (FR)
(74) Mandataire: Hubert, Philippe
(86) Numéro de dépôt international: FR9600782
(87) Numéro de publication internationale: WO9637285

(56) Documents cités:
- EP-A- 0 474 915
- EP-A- 0 628 305
- EP-A- 0 629 396
- WO-A-91/13961
- WO-A-92/06778
- WO-A-92/07543
- WO-A-95/13863
- DE-A- 4 319 699
- DE-A- 4 320 119
- GB-A- 1 284 456

## Description

La présente invention concerne une composition émulsionnante comprenant un mélange d'alkylpolyglycosides et au moins un alcool gras, qui peuvent être utilisées en tant que compositions auto-émulsionnables pour la préparation d'émulsions, des émulsions comprenant une telle composition émulsionnante, ainsi que les utilisations de celles-ci.

Il est bien connu de préparer des émulsions, notamment dans le domaine cosmétique et dermatologique, au moyen de tensioactifs non ioniques, du type hydrophile, obtenues par greffage d'une chaîne polyoxy-éthylée. Ces tensioactifs non ioniques, émulsionnants, peuvent se présenter sous la forme de compositions à base d'alcools, d'acides ou d'esters gras qui présentent l'avantage d'être auto-émulsionnables. On entend ici par "auto-émulsionnable" une composition qui permet d'obtenir une émulsion stable par simple mélange sous agitation modérée avec une phase aqueuse.

Les tensioactifs non ioniques comprenant une chaîne polyoxy-éthylée mentionnée ci-dessus présentent cependant l'inconvénient d'être plus ou moins irritants. Par ailleurs, il a pu être constaté que les émulsions préparées avec ces compositions auto-émulsionnantes ne présentaient une stabilité que sur une période de temps relativement courte.

En vue de remédier à ces inconvénients, la demanderesse a proposé dans la demande WO-A-92/06778 l'utilisation de compositions auto-émulsionnables à base d'alcools gras et d'alkylpolyglycosides ou alkylpolyosides. Des compositions auto-émulsionnables telles que décrites dans la demande précitée sont commercialisées par la société S.E.P.P.I.C. sous la marque MONTANOV®68. Celles-ci comportent un mélange d'alkylpoly-glycosides dont les chaînes grasses comprennent 16 et 18 atomes de carbone ainsi qu'un mélange d'alcools gras de même longueur de chaîne grasse.

Dans l'example 4 de ce document est divulguée une composition autoémulsionnable comprenant 64,3% d'alcools gras de C₁₂ à C₁₈ et 33,8% d'alkyl (C₁₂ à C₁₈) glucosides.

La Demanderesse a cependant constaté récemment que des compositions émulsionnantes utiles comme compositions auto-émulsionnables, du type MONTANOV®68, pouvaient conduire à l'obtention d'émulsions peu stables lorsqu'elles sont soumises à des températures basses, voire très basses, par exemple des températures inférieures à environ -20°C. Or, certains utilisateurs de ces compositions émulsionnantes souhaitent que les émulsions qu'ils préparent soient stables même à des températures de cet ordre.

Il est alors proposé par la présente invention des compositions émulsionnantes permettant l'obtention d'émulsions stables à des températures basses, par exemple des températures inférieures à -20°C. Ces compositions émulsionnantes, par ailleurs, ne présentent pas l'inconvénient des compositions à base de composés polyoxyéthylés mentionnés ci-dessus. Plus particulièrement, ces compositions émulsionnantes selon l'invention ne sont pas irritantes. En outre, les compositions émulsionnantes selon l'invention peuvent être utilisées comme compositions auto-émulsionnables.

La présente invention a donc pour premier objet une composition émulsionnante comprenant telle que definie dans la revendication 1.

Par "alcool gras de formule R'OH", on entend un alcool dérivé d'un acide gras, que ce soit un acide gras naturel ou qu'il soit obtenu par synthèse chimique. Il peut être notamment dérivé des acides octanoïque, décanoïque, dodécanoïque, tétradécanoïque, hexadecanoïque, octadécanoïque, eicosanoïque, docasanoïque, octadécénoïque, eicasénoïque, octodécadiénique, ou octadécatriénique.

Selon une caractéristique avantageuse, le rapport pondéral entre les alkylpolyglycosides de formule (I) et les alkylpolyglycosides de formules (II) et (III) est compris entre 0,5 et 1,5.

G₁, G₂, G₃, identiques ou différents, peuvent représenter le reste d'un sucre choisi parmi le dextrose, le saccharose, le fructose, le galactose, le maltose, le maltotriose, le lactose, la cellobiose, le mannose, le ribose, le dextrane, le thalose, l'allose, le xylose, le lévoglucosane, la cellulose et l'amidon. De préférence, le reste du sucre est un reste de glucose ou de fructose. Tout préférentiellement, G₁, G₂, G₃ sont identiques et représentent chacun le reste d'un glucose.

Les alkylpolyglycosides de formules (I), (II) et (III) ont une valeur de x₁, x₂ et x₃, identiques ou différents, comprise entre 1,1 et 2.

Selon un autre aspect avantageux de l'invention, la composition émulsionnante comprend un alkylpolyglycoside de formule (I) où R₁ est un radical aliphatique comprenant 12 atomes de carbone et un alkylpolyglycoside de formule (I) où R₁ est un radical aliphatique comprenant 14 atomes de carbone. Par ailleurs, R₂ et R₃ sont des radicaux alkyle, de sorte que R₂ représente un radical palmityle et R₃ représente un radical stéaryle. Le rapport pondéral entre les alkylpolyglycosides de formule (II) et les alkylpoly-glycosides de formule (III) est habituellement compris entre 10/90 et 90/10, de préférence entre 30/70 et 70/30.

Les alcools gras présents dans la composition émulsionnante comprennent un radical alkyle R' qui est de préférence saturé.

Selon un autre aspect, l'invention concerne également une émulsion comprenant au moins une phase aqueuse et une phase huile, ladite émulsion comprenant une composition émulsionnante selon l'invention, telle que définie ci-dessus.

La phase huile constitutive de l'émulsion peut être constituée par une huile choisie parmi les huiles suivantes :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de noisette, l'huile de palme, le beurre de karité, l'huile de noyau d'abricot, l'huile de calophyllum ;
- les huiles d'origine animale, telles que le perhydrosqualène ;
- les huiles minérales, telles que l'huile de paraffine, l'huile de vaseline, et les huiles minérales, notamment issues de coupes pétrolières, ayant un point d'ébullition compris entre 300 et 400°C ;
- les huiles synthétiques, telles que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les isoparaffines, et les huiles de silicone. Parmi ces dernières, on peut plus particulièrement citer les diméthylpolysiloxanes, méthylphénylpolysiloxanes, amines silicones amino-modifiés, les silicones modifiés par des acides gras, des silicones modifiés par des alcools, des silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyle.

Ces émulsions peuvent être préparées par dispersion à chaud, par exemple à une température comprise entre 50 et 80°C, dans de l'eau ou dans un solvant polaire, d'une composition émulsionnante selon l'invention ; cette dispersion étant réalisée par simple agitation, notamment mécanique, lente.

Lorsque l'émulsion comprend une huile telle que définie ci-dessus, celle-ci peut être fondue avec la composition émulsionnante selon l'invention, à une température comprise entre 50 et 80°C. Le mélange obtenu est ensuite dispersé dans l'eau ou le solvant polaire, porté à une température comprise entre 50 et 80°C.

De façon avantageuse, une émulsion selon l'invention comprend de 1 à 10 % en poids de la composition émulsionnante définie plus haut.

La composition émulsionnante selon l'invention peut être utilisée comme agent auto-émulsionnable pour la préparation d'une émulsion stable, notamment une émulsion stable à moins de -20°C, ladite composition émulsionnante et ladite émulsion étant telles que définies plus haut.

Elle a aussi pour objet l'utilisation des émulsions définies plus haut, par exemple ainsi préparées, dans l'hygiène du corps humain, ou animal, ainsi que les crèmes particulières selon les revendications 14 et 15, qui peuvent être ainsi préparées, pour la mise en oeuvre d'une méthode de traitement préventif ou symptomatique des affections cutanées du corps humain ou animal liées à l'exposition au soleil.

Les exemples qui suivent ont pour but d'illustrer la présente invention.

### Exemple 1 :

### Procédé de préparation d'une composition émulsionnante selon l'invention

On introduit dans un réacteur polyvalent une coupe d'alcools gras ayant la composition suivante (% en poids) :
- alcool laurique (C₁₂) 12,5 %
- alcool myristique (C₁₄) 17,5 %
- alcool palmitique (C₁₆) 29,0 %
- alcool stéarique (C₁₈) 41,0 %

On introduit également dans le réacteur du glucose de sorte que le rapport molaire entre les alcools gras et le glucose soit de 6:1. On fait ensuite réagir le glucose avec les alcools gras pendant 5 heures à une température comprise entre 100 et 105°C, en présence d'acide sulfurique en tant que catalyseur. La réaction est réalisée sous un vide partiel de 15 mm de mercure (2 kPa).

La composition obtenue comprend 75,5 % d'alcools gras libres en les teneurs suivantes (% en poids) :
- en C₁₂ 9,5
- en C₁₄ 13,0
- en C₁₆ 22,0
- en C₁₈ 31,0

Après réaction, on neutralise le catalyseur par une base. Les alcools en C₁₂ et C₁₄, présents dans le mélange réactionnel sont partiellement éliminés par distillation sur un évaporateur à film mince. On ajoute dans le milieu réactionnel des alcools gras comprenant 16 et 18 atomes de carbone en vue d'obtenir la composition désirée.

La composition obtenue comprend :
- alcools gras libres (% en poids) :
   - en C₁₂ ≤ 1, 2
   - en C₁₄ ≤ 2,0
   - en C₁₆ 26,0
   - en C₁₈ 36,0
- alkylpolyglucosides (% en poids) :
   - en C₁₂ 5,76
   - en C₁₄ 7,44
   - en C₁₆ 9,60
   - en C₁₈ 13,2

### Exemple 2 :

### Procédé de préparation d'une composition émulsionnante selon l'invention

En vue de préparer une composition émulsionnante selon l'invention, on a mélangé une composition 2 et une composition 3, chacune à base d'un mélange d'alkylpolyglucosides et d'alcools gras libres.

La composition 2 a été préparée selon le procédé décrit dans la demande internationale WO-92/06778. Cette composition 2 comprenait (% en poids) :
- alkylpolyglucosides en C₁₂ 10,0 %
- alkylpolyglucosides en C₁₄ 10,0 %
- alcool cétylstéarilique 80,0 %

La composition 3 a été préparée par réaction du glucose sur un mélange approprié d'alcools, selon le procédé décrit dans l'exemple 3 de la demande internationale PCT/FR/94/01336, déposée le 16 novembre 1994, au nom de S.E.P.P.I.C. Plus précisément, cette composition 3 comprenait (% en poids) :
- alkylpolyglucosides en C₁₂ 14,4 %
- alkylpolyglucosides en C₁₄ 18,6 %
- alkylpolyglucosides en C₁₆ 09,0 %
- alkylpolyglucosides en C₁₈ 18,0 %
- alcool laurique 03,0 %
- alcool myristique 04,0 %
- alcool palmitique 06,0 %
- alcool stéarique 27,0 %

Les compositions 2 et 3 se présentaient sous forme solide. On les a fait fondre ensemble à 70°C, dans des proportions appropriées pour obtenir une composition émulsionnante selon l'invention, identique dans ses constituants et leurs proportions relatives à celle obtenue dans l'exemple 1 ci-dessus.

### Exemple 3 :

### Crème solaire

On prépare une crème solaire comprenant les composés suivants (% en poids) :
* phase A) :
   - composition émulsionnante de l'exemple 2 5,0 %
   - diméthicone 5,0 %
   - cyclométhicone 10,0 %
   - benzophénone-3 0,5 %
   - octyl paraméthoxycinnamate 5,0 %
* phase B) :
   - sépicide HB⁽¹⁾ 0,5 %
   - sépicide Cl⁽²⁾ 0,2 %
   - parfums 0,1 %
* phase C) :
   eau q.s.p. 100 %

(1) : conservateur commercialisé par la société S.E.P.P.I.C.
(2) : conservateur commercialisé par la société S.E.P.P.I.C.

La crème solaire est obtenue de la manière suivante :
- on fond la phase A) à 70°C ;
- on chauffe la phase C) à 75°C ;
- on mélange la phase A) avec la phase C) sous agitation modérée pour former une émulsion ;
- on introduit la phase B) dans l'émulsion formée, lorsque la température de cette dernière atteint 40°C ;
- on ajuste le pH à 6, si nécessaire.

L'émulsion obtenue a l'aspect d'une crème blanche brillante. Sa viscosité est de 25 000 mPa.s (Brookfield LVF, module IV, six tours/min.).

En vue d'apprécier la stabilité au froid de l'émulsion obtenue, on l'a soumise au test suivant :
- on a conditionné l'émulsion dans deux flacons de verre de 30 ml. L'un de ces flacons est congelé à -26°C tandis que l'autre flacon (émulsion témoin) est conservé à température ambiante. L'émulsion contenue dans ce dernier flacon sert de référence.

L'émulsion testée est placée à -26°C pendant 18 heures puis décongelée spontanément à température ambiante pendant 6 heures. L'émulsion-test et l'émulsion-témoin font alors l'objet d'un examen visuel comparatif et sont notées de la façon suivante :
- 0 : l'émulsion-test est comparable à l'émulsion-témoin quant à son aspect homogène, lisse et brillant.
- X : l'émulsion-test présente une surface mate et moins lisse que l'émulsion-témoin.
- XX : l'émulsion-test présente une surface nettement granuleuse* avec perte de brillance, aspect caractéristique et facilement identifiable même sans référence à l'émusion-témoin.
- XXX : l'émulsion-test est déstructurée et on retrouve en surface une exsudation d'eau.
   * L'aspect est granuleux mais on ne note pas la présence physique de grains lorsqu'on étale l'émulsion ; il s'agit uniquement d'un contrôle et d'une appréciation visuels.

La crème solaire obtenue selon le procédé décrit ci-dessus, soumise au test froid, tel que défini plus haut, a été notée.

### Exemple 4 :

### Crème aux huiles végétales

On a préparé une crème aux huiles végétales comprenant les composés suivants (% en poids) :
* phase A) :
   - composition émulsionnante de l'exemple 2 5,0 %
   - huile de jojoba 5,0 %
   - huile de noisette 5,0 %
   - huile d'amande douce 10,0 %
   - DL α-tocophérol 0,05 %
* phase B) :
   - sépicide HB⁽¹⁾ 0,3 %
   - sépicide Cl⁽²⁾ 0,2 %
   - parfums 0,4 %
* phase C :
   - eau q.s.p. 100 %

⁽¹⁾ : conservateur commercialisé par la société S.E.P.P.I.C.
⁽²⁾ : conservateur commercialisé par la société S.E.P.P.I.C.

La crème aux huiles végétales est préparée de la manière suivante
- on fond la phase A) à 70°C ;
- on chauffe la phase C) à 75°C ;
- on mélange la phase A) avec la phase C) sous agitation modérée pour former une émulsion ;
- on introduit la phase B) dans l'émulsion formée, lorsque la température de cette dernière a atteint 40°C environ ;
- on ajuste le pH à 6, si nécessaire.

L'émulsion obtenue a l'aspect d'une crème ivoire brillante, sa viscosité est de 23 000 mPa.s (Brookfield LVF, module IV, six tours/min.).

On soumet cette crème aux huiles végétales au test froid décrit dans l'exemple 3 ci-dessus. L'examen visuel de la composition soumise à ce test a conduit à noter celle-ci : 0

### Exemple 5 :.

On a préparé différentes émulsions comprenant (% en poids) :
- composition émulsionnante à base d'alkylpolyglucosides et d'alcools gras 5,0 %
- huile 20 %
- eau, q.s.p. 100 %

La composition émulsionnante mise en oeuvre était soit celle de l'exemple 2 (composition I), soit la composition émulsionnante 2 définie dans l'exemple 2 ci-dessus (composition II).

Les huiles utilisées étaient les suivantes :
- PRIMOL 352 : huile de paraffine commercialisée par la société ESSO
- LANOL 1688 : cétéaryle octanoate, commercialisé par la société S.E.P.P.I.C.
- huile d'amandes douces (HAD) ;
- DC 200/350 : diméthicone commercialisé par la société Dow Corning.

Les émulsions obtenues ont été soumises au test froid décrit dans l'exemple 3. Les résultats obtenus figurent dans le tableau 1 ci-dessous :

**Tableau 1 :**

| Composition émulsionnante | Huile | | | |
|---|---|---|---|---|
| | PRIMOL 352 | LANOL 1688 | HAD | DC 200/350 |
| I | 0 | 0 | 0 0 | |
| II | XXX | XXX | XXX | XXX |

Les résultats obtenus montrent que les compositions selon l'invention sont nettement plus stables lorsqu'elles sont soumises à de basses températures que des émulsions selon l'art antérieur.

## Revendications

1. Composition émulsionnante comprenant de 10 à 40 % en poids d'un mélange d'alkylpolyglycosides et de 90 à 60 % en poids d'au moins un alcool gras de formule R'OH, où R' est un radical alkyle, saturé ou insaturé, ayant de 12 à 18 atomes de carbone, caractérisée
en ce que le mélange d'alkylpolyglycosides comprend :
(i) au moins un alkylpolyglycoside de formule (I)
R₁O(G₁)X₁ (I)
dans laquelle R₁ est un radical aliphatique, linéaire ou ramifié, ayant 12 ou 14 atomes de carbone, G₁ est le reste d'un sucre, X₁ est compris entre 1 et 5 ;
(ii) un alkylpolyglycoside de formule (II)
R₂O (G₂) X₂ (II)
dans laquelle R₂ est un radical aliphatique, linéaire ou ramifié, ayant 16 atomes de carbone, G₂ est le reste d'un sucre, X₂ est compris entre 1 et 5 ;
(iii) un alkylpolyglycoside de formule (III)
R₃O (G₃) X₃ (III)
dans laquelle R₃ est un radical aliphatique, linéaire ou ramifié, ayant 18 atomes de carbone, G₃ est le reste d'un sucre et, X₃ est compris entre 1 et 5,
le rapport pondéral entre les alkylpolyglycosides de formule (I) et les alkylpolyglycosides de formule (II) et (III) étant compris entre 0,4 et 5 ; et en ce que en le poids du mélange d'alcools gras comprend:
(i) au moins un alcool gras comprenant 12 ou 14 atomes de carbone;
(ii) un alcool gras comprenant 16 atomes de carbone et un alcool gras comportant 18 atomes de carbone,
le rapport pondéral entre les alcools gras comprenant 12 ou 14 atomes de carbone et les alcools gras comprenant 16 et 18 atomes de carbone étant compris entre 0,03 et 0,1.

2. Composition selon la revendication 1, caractérisée en ce que le rapport pondéral entre les alkylpolyglycosides de formule (I) et les alkylpolyglycosides de formules (II) et (III) est compris entre 0,5 et 1,5.

3. Composition selon l'une quelconque des revendications 1 ou 2, dans laquelle G₁, G₂, G₃ représentent indépendamment l'un de l'autre un reste fructose ou glucose.

4. Composition émulsionnante selon la revendication 3, caractérisée en ce que G₁, G₂ et G₃ représentent un reste glucose.

5. Composition selon l'une quelconque des revendications 1 à 4, caractérisée en ce que X₁, X₂ et X₃, identiques ou différents, sont chacun compris entre 1,1 et 2.

6. Composition selon l'une quelconque des revendications 1 à 5, caractérisée en ce qu'elle comprend un alkylpolyglycoside de formule (I) où R₁ est un radical aliphatique comprenant 12 atomes de carbone et un alkylpolyglycoside de formule (I) où R₁ est un radical aliphatique comprenant 14 atomes de carbone.

7. Composition selon l'une quelconque des revendications 1 à 6, caractérisée en ce que R₂ et R₃ sont des radicaux alkyle et notamment en ce que R₂ représente un radical palmityle et R₃ représente un radical stéaryle.

8. Composition selon l'une quelconque des revendications 1 à 7, dans laquelle le rapport pondéral entre l'alkylpolyglycoside de formule (II) et l'alkylpolyglycoside de formule (III) est compris entre 10/90 et 90/10 et de préférence entre 30/70 et 70/30.

9. Composition selon l'une quelconque des revendications 1 à 8, caractérisée en ce que le mélange d'alcools gras comporte un alcool gras comprenant 12 atomes de carbone et un alcool gras comprenant 14 atomes de carbone.

10. Composition selon la revendication 9, comprenant :
au plus 1,2 % d'alcools gras libres en C₁₂ ;
au plus 2,0 % d'alcools gras libres en C₁₄ ;
26 % d'alcools gras libres en C₁₆;
36 % d'alcools gras libres en C₁₈ ;
5,7 % d'alkylpolyglucosides en C₁₂;
7,44 % d'alkylpolyglucosides en C₁₄ ;
9,60 % d'alkylpolyglucosides en C₁₆ ;
13,20 % d'alkylpolyglucosides en C₁₈.

11. Une émulsion comprenant au moins une phase aqueuse et une phase huile, caractérisée en ce qu'elle comprend une composition émulsionnante selon l'une des revendications 1 à 10.

12. Emulsion selon la revendication 11, caractérisée en ce que la phase huile comprend au moins un composé choisi parmi les huiles de silicone, les huiles d'origine végétale et l'huile de paraffine.

13. Emulsion selon l'une des revendications 11 et 12, caractérisée en ce qu'elle comprend de 1 à 10 % en poids de la composition émulsionnante selon l'une quelconque des revendications 1 à 10.

14. Crème solaire contenant :
- la composition émulsionnante selon
- la revendication 10 5 %
- diméthicone 5 %
- cyclométhicone 5 %
- benzophénone-3 0,5 %
- octylparaméthoxycinnamate 5 %
- sépicide HB 0,5 %
- sepicide Cl 0,2 %
- parfums 0,1 %
- eau q.s.p. 100 %

15. Crème aux huiles végétales contenant :
* phase A) :
- composition émulsionnante selon la revendication 10 5,0 %
- huile de jojoba 5,0 %
- huile de noisette 5,0 %
- huile d'amande douce 10,0 %
- DL α-tocophérol 0,05 %
* phase B) :
- sépicide HB 0,3 %
- sépicide Cl 0,2 %
- parfums 0,4 %
* phase C :
- eau q.s.p. 100 %

16. Utilisation des émulsions selon l'une quelconque des revendications 11 à 15 dans l'hygiène du corps humain ou animal.

17. Crème selon l'une des revendications 14 ou 15 pour la mise en oeuvre d'une méthode de traitement préventif ou curatif des affections cutanées du corps humain ou animal liées à l'exposition au soleil.

## Patentansprüche

1. Emulgierende Zusammensetzung, die 10 bis 40 Gew.-% eines Alkylpolyglykosidgemischs und 90 bis 60 Gew.-% wenigstens eines Fettalkohols mit der Formel R'OH, worin R' einen gesättigten oder ungesättigten Alkylrest mit 12 bis 18 Kohlenstoffatomen bedeutet, umfasst, **dadurch gekennzeichnet, dass** das Alkylpolyglykosidgemisch
a) mindestens ein Alkylpolyglykosid mit der Formel (I)
R₁O(G₁)X₁ (I),
worin R₁ einen geradkettigen oder verzweigten aliphatischen Rest mit 12 oder 14 Kohlenstoffatomen und G₁ einen Zuckerrest bedeutet und X₁ 1 bis 5 beträgt,
b) ein Alkylpolyglykosid mit der Formel (II)
R₂O(G₂)X₂ (II),
worin R₂ einen geradkettigen oder verzweigten aliphatischen Rest mit 16 Kohlenstoffatomen und G₂ einen Zuckerrest bedeutet und X₂ 1 bis 5 beträgt, und
c) ein Alkylpolyglykosid mit der Formel (III)
R₃O(G₃)X₃ (III),
worin R₃ einen geradkettigen oder verzweigten aliphatischen Rest mit 18 Kohlenstoffatomen und G₃ einen Zuckerrest bedeutet und X₃ 1 bis 5 beträgt, enthält, wobei das Gewichtsverhältnis von Alkylpolyglykosiden mit der Formel (I) zu Alkylpolyglykosiden mit den Formeln (II) und (III) 0,4 bis 5 beträgt, **und dass** das Fettalkoholgemisch
a) mindestens einen Fettalkohol mit 12 oder 14 Kohlenstoffatomen und
b) einen Fettalkohol mit 16 Kohlenstoffatomen und einen Fettalkohol mit 18 Kohlenstoffatomen enthält,
wobei das Gewichtsverhältnis von Fettalkoholen mit 12 oder 14 Kohlenstoffatomen zu Fettalkoholen mit 16 und 18 Kohlenstoffatomen 0,03 bis 0,1 beträgt.

2. Zusammensetzung nach Anspruch 1, **dadurch gekennzeichnet, dass** das Gewichtsverhältnis von Alkylpolyglykosiden mit der Formel (I) zu Alkylpolyglykosiden mit den Formeln (II) und (III) 0,5 bis 1,5 beträgt.

3. Zusammensetzung nach Anspruch 1 oder 2, wobei G₁, G₂ und G₃ unabhängig voneinander einen Fructose- oder Glucoserest bedeuten.

4. Emulgierende Zusammensetzung nach Anspruch 3, **dadurch gekennzeichnet, dass** G₁, G₂ und G₃ einen Glucoserest bedeuten.

5. Zusammensetzung nach einem der Ansprüche 1 bis 4, **dadurch gekennzeichnet, dass** X₁, X₂ und X₃, die gegebenenfalls verschieden sind, jeweils 1,1 bis 2 betragen.

6. Zusammensetzung nach einem der Ansprüche 1 bis 5, **dadurch gekennzeichnet, dass** sie ein Alkylpolyglykosid mit der Formel (I), worin R₁ einen aliphatischen Rest mit 12 Kohlenstoffatomen bedeutet, und ein Alkylpolyglykosid mit der Formel (I), worin R₁ einen aliphatischen Rest mit 14 Kohlenstoffatomen bedeutet, umfasst.

7. Zusammensetzung nach einem der Ansprüche 1 bis 6, **dadurch gekennzeichnet, dass** R₂ und R₃ Alkylreste bedeuten und insbesondere R₂ einen Palmitylrest und R₃ einen Stearylrest bedeutet.

8. Zusammensetzung nach einem der Ansprüche 1 bis 7, wobei das Gewichtsverhältnis von Alkylpolyglykosid mit der Formel (II) zu Alkylpolyglykosid mit der Formel (III) 10/90 bis 90/10 und vorzugsweise 30/70 bis 70/30 beträgt.

9. Zusammensetzung nach einem der Ansprüche 1 bis 8, **dadurch gekennzeichnet, dass** das Fettalkoholgemisch einen Fettalkohol mit 12 Kohlenstoffatomen und einen Fettalkohol mit 14 Kohlenstoffatomen enthält.

10. Zusammensetzung nach Anspruch 9, umfassend:
- höchstens 1,2 % freie C₁₂-Fettalkohole,
- höchstens 2,0 % freie C₁₄-Fettalkohole,
- 26 % freie C₁₆-Fettalkohole,
- 36 % freie C₁₈-Fettalkohole,
- 5,7 % C₁₂-Alkylpolyglucoside,
- 7,44 % C₁₄-Alkylpolyglucoside,
- 9,60 % C₁₆-Alkylpolyglucoside,
- 13,20 % C₁₈-Alkylpolyglucoside.

11. Emulsion, die mindestens eine wässrige Phase und eine ölige Phase umfasst, **dadurch gekennzeichnet, dass** sie eine emulgierende Zusammensetzung nach einem der Ansprüche 1 bis 10 enthält.

12. Emulsion nach Anspruch 11, **dadurch gekennzeichnet, dass** die ölige Phase mindestens eine Verbindung enthält, die aus Siliconölen, Ölen pflanzlichen Ursprungs und Paraffinöl ausgewählt ist.

13. Emulsion nach Anspruch 11 oder 12, **dadurch gekennzeichnet, dass** sie 1 bis 10 Gew.-% der emulgierenden Zusammensetzung nach einem der Ansprüche 1 bis 10 enthält.

14. Sonnenschutzcreme, enthaltend:
- emulgierende Zusammensetzung nach Anspruch 10 5 %,
- Dimethicon 5 %,
- Cyclomethicon 5 %,
- Benzophenon-3 0,5 %,
- Octylparamethoxycinnamat 5 %,
- Sépicide HB 0,5 %,
- Sépicide Cl 0,2 %,
- Parfüms 0,1 %,
- Wasser auf 100 %.

15. Pflanzenölcreme, enthaltend:
* Phase A:
- emulgierende Zusammensetzung nach Anspruch 10 5,0 %,
- Jojobaöl 5,0 %,
- Nussöl 5,0 %,
- süßes Mandelöl 10,0 %,
- DL-α-Tocopherol 0,05 %,
* Phase B :
- Sépicide HB 0,3 %,
- Sépicide Cl 0,2 %,
- Parfüms 0,4 %,
* Phase C:
- Wasser auf 100 %.

16. Verwendung von Emulsionen nach einem der Ansprüche 11 bis 15 bei der Hygiene von Mensch oder Tier.

17. Creme nach Anspruch 14 oder 15 für die Durchführung eines präventiven oder kurativen Verfahrens zur Behandlung von durch Sonneneinstrahlung hervorgerufenen Hautleiden von Mensch oder Tier.

## Claims

1. An emulsifying composition comprising from 10 to 40% by weight of a mixture of alkylpolyglycosides and from 90 to 60% by weight of at least one fatty alcohol of formula R'OH, wherein R' is a saturated or unsaturated alkyl radical having from 12 to 18 carbon atoms, characterised :
- in that the mixture of alkylpolyglycosides comprises:
(i) at least one alkylpolyglycoside of formula (I) :
R₁O(G₁)X₁ (I)
in which R₁ is a linear or branched aliphatic radical having 12 or 14 carbon atoms, G₁ is a sugar residue and X₁ is between 1 and 5 ;
(ii) an alkylpolyglycoside of formula (II) :
R₂O(G₂)X₂ (II)
in which R₂ is a linear or branched aliphatic radical having 16 carbon atoms, G₂ is a sugar residue, X₂ is between 1 and 5 ; and
(iii) an alkylpolyglycoside of formula (III) :
R₃O(G₃)X₃ (III)
in which R₃ is a linear or branched aliphatic radical having 18 carbon atoms, G₃ is a sugar residue and X₃ is between 1 and 5 ; the ratio by weight of the alkylpolyglycosides of formula (I) to the alkylpolyglycosides of formulae (II) and (III) being between 0.4 and 5 ;
- and in that the mixture of fatty alcohols comprises:
(i) at least one fatty alcohol comprising 12 or 14 carbon atoms; and
(ii) a fatty alcohol comprising 16 carbon atoms and a fatty alcohol comprising 18 carbon atoms ;
the ratio by weight of the fatty alcohols comprising 12 or 14 carbon atoms to the fatty alcohols comprising 16 to 18 carbon atoms being between 0.03 and 0.1.

2. The composition according to claim 1, characterised in that the ratio by weight of the alkylpolyglycosides of formula (I) to the alkylpolyglycosides of formulae (II) and (III) is between 0.5 and 1.5.

3. The composition according to any one of claims 1 or 2, in which G₁, G₂, and G₃ independently of each other represent a fructose or glucose residue.

4. The emulsifying composition according to claim 3, characterised in that G₁, G₂, and G₃ represent a glucose residue.

5. The composition according to any one of claims 1 to 4, characterised in that X₁, X₂ and X₃, which are identical or different, are each between 1.1 and 2.

6. The composition according to any one of claims 1 to 5, characterised in that it comprises an alkylpolyglycoside of formula (I) wherein R₁ is an aliphatic radical comprising 12 carbon atoms and an alkylpolyglycoside of formula (I) wherein R₁ is an aliphatic radical comprising 14 carbon atoms.

7. The composition according to any one of claims 1 to 6, characterised in that R₂ and R₃ are alkyl radicals, and notably in that R₂ represents a palmityl radical and R₃ represents a stearyl radical.

8. The composition according to any one of claims 1 to 7, in which the ratio by weight of the alkylpolyglycoside of formula (II) and the alkylpolyglycoside of formula (III) is between 10/90 and 90/10, and preferably between 30/70 and 70/30.

9. The composition according to any one of claims 1 to 8, characterised in that the mixture of fatty alcohols comprises a fatty alcohol comprising 12 carbon atoms and a fatty alcohol comprising 14 carbon atoms.

10. The composition according to claim 9, comprising :
at most 1.2% of free C₁₂ fatty alcohols ;
at most 2.0% of free C₁₄ fatty alcohols ;
26% of free C₁₆ fatty alcohols ;
36% of free C₁₈ fatty alcohols ;
5.7% of C₁₂ alkylpolyglucosides ;
7.44% of C₁₄ alkylpolyglucosides ;
9.60% of C₁₆ alkylpolyglucosides ; and
13.20% of C₁₈ alkylpolyglucosides.

11. An emulsion comprising at least one aqueous phase and one oil phase, characterised in that it comprises an emulsifying composition according to one of claims 1 to 10.

12. The emulsion according to claim 11, characterised in that the oil phase comprises at least one compound selected from silicone oils, oils of plant origin, and liquid paraffin.

13. The emulsion according to one of claims 11 and 12, characterised in that it comprises from 1 to 10% by weight of the emulsifying composition according to any one of claims 1 to 10.

14. A sun cream containing :
- the emulsifying composition according to claim 10 5%,
- dimethicone 5%,
- cyclomethicone 5%,
- 3-benzophenone 0.5%,
- octyl *para*-methoxycinnamate 5%,
- sépicide HB 0.5%,
- sépicide C1 0.2%,
- fragrances 0.1 %, and
- water q.s. for 100%.

15. A plant oil cream containing :
* phase A) :
- emulsifying composition according to claim 10 5.0%,
- jojoba oil 5.0%,
- hazelnut oil 5.0%,
- sweet almond oil 10.0%,
- DL-α-tocopherol 0.05% ;
* phase B) :
- sépicide HB 0.3%,
- sépicide C1 0.2%,
- fragrances 0.4%
* phase C) :
- water q.s. for 100%.

16. Use of the emulsions according to any one of claims 11 to 15 in the hygiene of the human or animal body.

17. The cream according to one of claims 14 or 15 for the implementation of a method of preventative or curative treatment of cutaneous affections of the human or animal body related to exposure to sunlight.
